# EUROPEAN PATENT SPECIFICATION

(11) **EP 4 637 853 B1**
(45) Date of publication and mention of the grant of the patent: **19.08.2026**
(21) Application number: 23828750.2
(22) Date of filing: 14.12.2023
(51) Int. Cl.: A61L 26/00, C08G 18/73, C08G 18/50, C08G 18/48, C08G 18/16, C08G 18/10, A61L 27/52, A61L 27/54

(54) **METHOD OF MANUFACTURING A HYDROGEL USEFUL FOR PREVENTING POST-SURGICAL ADHESIONS**
VERFAHREN ZUR HERSTELLUNG EINES HYDROGELS GEEIGNET ZUR VERHINDERUNG POSTCHIRURGISCHER VERKLEBUNGEN
PROCÉDÉ DE FABRICATION D'UN HYDROGEL UTILE POUR PRÉVENIR LES ADHÉRENCES POST-CHIRURGICALES

(30) Priority: 20.12.2022 EP 22214907; 02.02.2023 EP 23154695; 05.09.2023 EP 23195323
(43) Date of publication of application: 29.10.2025
(73) Proprietor: Covestro Deutschland AG, 51373 Leverkusen (DE)
(72) Inventor: KORTENBREDE, Lana, 50739 Köln (DE); HECKROTH, Heike, 51519 Odenthal (DE); SUETTERLIN, Jan, 51061 Köln (DE)
(74) Representative: Levpat
(86) International application number: PCT/EP2023/085794
(87) International publication number: WO 2024/132836

(56) References cited:
- DE-U1- 202018 004 305
- US-A1- 2009 191 145
- US-A1- 2012 244 107
- DATABASE WPI Week 201582, Derwent World Patents Index; AN 2015-616646, XP002809463

## Description

The present invention relates to a method of manufacturing a hydrogel comprising reacting: A) An isocyanate-terminated prepolymer obtained by the reaction of at least: A1) A diisocyanate with a molar mass of 140 to 278 g/mol; A2) A polyalkylene oxide with an ethylene oxide content of ≥ 50 weight-%, calculated from the total weight of the polyalkylene oxide; B) A polyamine and C) Water. The invention also relates to a hydrogel obtainable by such a method, the use of such a hydrogel in surgery, as a post-surgical adhesion barrier and in delayed-release formulations.

Adhesions are among the most frequent complications after surgical interventions in the abdominal and pelvic regions. Adhesions are fibrous bands which generally form within the first seven days after an operation, in the course of the healing process. They cause tissues and organs which are normally separated from one another to grow together, which can give rise to a multiplicity of complications such as, for example, chronic pain, infertility or a lifethreatening intestinal occlusion. Products able to reduce the formation of adhesions have been developed in recent years to avoid such complications.

Hydrogels have been used as adhesion barriers as well as other materials. Hydrogels are hydrophilic crosslinked polymers that do not dissolve in it. Crosslinks can be formed by covalent chemical bonds or non-covalent interactions such as electrostatic, hydrophobic or dipole-dipole interactions between individual segments of polymer chains, building a three-dimensional network. These networks swell in water up to an equilibrium volume with substantial shape retention. Desired properties of hydrogels are specifically targetable via the choice of monomers used for polymer construction, the type of crosslinking and the crosslink density.

DE 20 2018 004305 U1 discloses a polyurethane hydrogel obtained by the reaction of at least the components a) a hydrophilic aliphatic and/or cycloaliphatic polyisocyanate prepolymer with a monomeric diisocyanate content of less than 1 weight-% with b) a compound having at least one amino group in the presence of c) water. Compound b) has a primary amino group and a carboxylic acid group. Salts of weak acids and zwitterions are included as well. The polyurethane hydrogel may be part of a wound dressing.

US 2012/244107 A1 relates to a hydrogel based on polyurethane or polyurethaneurea, having hydrolyzable functional groups in the polymer chain and obtained by reaction of A) a polyisocyanate prepolymer having hydrolyzable groups in the polymer chain, B) water, C) optionally hydroxyl-amino compounds having at least one tertiary amino group and at least three hydroxyl groups, D) optionally catalysts, and E) optionally auxiliary and addition agents. Said polyisocyanate prepolymer A) is obtained by reaction of A1) a polyisocyanate with A2) a polyol having hydrolyzable groups in the polymer chain. Said polyol A2) comprises polyesters and/or polyetheresters that are liquid at room temperature and have a DIN 53019 shear viscosity at 23° C in the range of 200 to 8000 mPas. Also disclosed is an adhesion barrier comprising the claimed hydrogel.

US 4,795,764 concerns a poly(oxyalkylene) poly(aliphatic isocyanate) prepolymer and polyurea polymer derived therefrom by reaction with polyamine. Discussed is a solution comprising polyamine reactant, low moisture sensitive poly(oxyalkylene) poly(aliphatic isocyanate) prepolymer the oxyalkylene portion of which contains sufficient oxyethylene units to render the prepolymer hydrophilic and water-soluble, and a water-soluble or -dispersible organic solvent which is free of active hydrogen atoms and in which said prepolymer is dissolved, the isocyanate moieties of said prepolymer being reactive with said polyamine. Further disclosed is an aqueous solution comprising the aforementioned solution and water as the major component of the aqueous solution.

WO 2003/050276 A1 discloses a polyurethane-hydrogel composition having an immobilized biologic, said composition being prepared from a process comprising the steps of (a) admixing at least one prepolymer and at least one water-soluble crosslinker in aqueous solvent and in the substantial absence of organic solvent to form a polyurethane-hydrogel mixture, said prepolymer being prepared from at least one water-soluble polyol and at least one isocyanate; and (b) contacting said mixture with a biologic to immobilize the biologic in said mixture to form a composition having an immobilized biologic, wherein said composition is substantially polymerized, is transparent, and has an effective number-average molecular weight between crosslinks.

US 2013/060216 A1 relates to a hydrogel matrix with improved adhesive characteristics. US 2013/204217 A1 relates to a hydrogel matrix having increased absorption capacity for liquids.

The biodegradable hydrogels in the art for use as adhesion barriers suffer from the drawback that some of the components needed for their formation have high viscosities. This limits their application to body surfaces via spraying.

The present invention has the object of providing a hydrogel which is suitable as a post-surgical adhesion barrier having enhanced biodegradability and which can be applied by spraying. This object has been achieved by a method according to claim 1 and a hydrogel according to claim 12. The invention also relates to a hydrogel for use according to claims 13, 14 and 15. Advantageous embodiments are the subject of the dependent claims. They can be combined freely unless the context clearly indicates otherwise.

Accordingly, a method of manufacturing a hydrogel comprises reacting the components:
A) An isocyanate-terminated prepolymer obtained by reacting a mixture comprising: A1) a diisocyanate with a molar weight of 140 to 278 g/mol; A2) a polyalkylene oxide with an ethylene oxide content of ≥ 50 weight-%, calculated from the total weight of the polyalkylene oxide; B) A polyamine having a molecular weight of ≥ 200 g/mol and C) water.

Examples for suitable polyisocyanates A1) include methylene diphenyl diisocyanate (MDI), methylene dicyclohexyl diisocyanate (H12-MDI), polymeric MDI, toluylene diisocyanate (TDI), xylylene diisocyanate (XDI), pentamethylene diisocyanate (PDI), hexamethylene diisocyanate (HDI), isophorone diisocyanate (IPDI) and a mixture comprising at least two of the aforementioned polyisocyanates.

The polyalkylene oxide A2) and/or the polyamine B) further comprise ester groups, expressed as -C(=O)-O-, in an amount of > 0.51 weight-%, based on the total weight of polyalkylene oxide A2) or polyamine B), respectively. It is preferred that the polyalkylene oxide A2) comprises ester groups of > 0.51 weight-%, based on the total weight of polyalkylene oxide A2), while the polyamine B) does not comprise ester groups. Preferably the ester group content in A2) and/or B is ≤ 5.1 weight-%. Further preferred ranges are > 0.51 weight-% to ≤ 5 weight-%, > 0.51 weight-% to ≤ 4.5 weight-% or ≥ 1 weight% to ≤ 4.5 weight-%, more preferred ≥ 1.2 weight-% to ≤ 3 weight-%.

Ester groups, which include diester block in the context of the present invention, may be introduced into the polyalkylene A2) by adding a cyclic ester dimer and/or polyester to a reaction mixture comprising a starter and alkylene oxide(s). The same considerations apply to the synthesis of ester group-containing polyamines B).

Examples for suitable cyclic ester dimers include dilactide (providing "lactide groups") and glycolide (providing "glycolide groups"). Examples for suitable polyesters include polylactic acid and polyglycolic acid. Mixtures of two or more of the aforementioned compounds can also be used.

The ester group contents for polyether polyols may be calculated by the following equation: $Ester {content}_{Polyol} = \frac{{m}_{cyclic ester} ∗ 44 ∗ f}{\frac{{MW}_{cyclic ester}}{{m}_{EO} + {m}_{PO} + {m}_{Dilactid} + {m}_{Starter}}} ∗ 100$

| | |
|---|---|
| *m_{cyclic ester}=* Total mass of added dilactide | *MW_{cyclic ester}=* molecular weight of cyclic ester (for all conducted experiments = 144 g/mol) |
| 44=molar mass of -O-C=O | *m_{EO}; m_{PO}; m_{Dilactid}; M_{Starter}=* total mass of EO/PO/dilactide/starter |
| *f* = factor for the amount of ester units per cyclic ester (for all conducted experiments =2) | 100=factor for percentage |

The ester group contents for prepolymers may be calculated by the following equation: $\begin{matrix}Ester {content}_{Prepolymer} \\ = \frac{\frac{{m}_{polyol} ∗ ester {content}_{polyol}}{100}}{\left(\frac{{m}_{polyol}}{eq . wght . polyol}∗{MW}_{Diisocyanate}\right) + {m}_{polyol}} ∗ 100\end{matrix}$

| | |
|---|---|
| *m_{polyol}=* Total mass of added polyol | *eq. wght. polyol=* equivalent weight of the polyol |
| *ester content_{polyol}=* calculated as in eq. 1.1 | *MW_{Diisocyanate}=* molar mass of used diisocyanate (for all conducted experiments =168.2 g/mol) |

The polyamine B) preferably has a molecular weight of ≥ 300 g/mol and ≤ 10000 g/mol as determined by amine number determination according to DIN EN ISO 1877-2. Molecular weight may be calculated as described in equation 1.3, wherein F is the NH₂ functionality, AN is the determined amine number according to DIN EN ISO 1877-2 and 56100 as molecular weight of potassium hydroxide multiplied with 100. $Molecular weight = \frac{F ⋅ 56100}{AN}$

The average OH functionality of polyalkylene oxide A2) is greater than 2 and/or the average combined primary and secondary amine functionality of polyamine B) is greater than 2. Possible combinations include a trifunctional polyalkylene oxide A2) and a difunctional polyamine B), a trifunctional polyalkylene oxide A2) and a trifunctional polyamine B) and a difunctional polyalkylene oxide A2) and a trifunctional polyamine B).

The combined components A) and B) are present in an amount of ≥ 11.5 weight-% ("solids content"), based on the total weight of components A), B) and C). Preferred is an amount of > 11.5 weight-% to ≤ 50 weight-%, more preferred > 13 weight-% to ≤ 40 weight-%, even more preferred ≥ 13.2 weight-% to ≤ 20 weight-%.

It has been found that the hydrogels obtainable by the method according to the invention have the combined properties of a useful biodegradability profile, an adhesion to internal organs and a usefully fast time for their formation. In preferred embodiments it has been found that the biodegradability, determined via loss of mechanical properties in accordance with the method laid out in the experimental section of this disclosure, is > 6 days to ≤ 28 days. In preferred embodiments it has been found that the adhesion to internal organs is good or very good, determined in accordance with the method laid out in the experimental section. In preferred embodiments it has been found that the formation time for the hydrogels, determined in accordance with the method laid out in the experimental section, is ≤ 60 seconds.

The reaction mixture for obtaining the hydrogels and the thus obtained hydrogels may be free from urethane group formation catalysts.

In one embodiment the NCO/NH molar ratio between prepolymer A) and polyamine B) is > 1.0 to ≤ 1.4. Preferred is a ratio of ≥ 1.1 to ≤ 1.3.

In another embodiment the prepolymer A) and/or the polyamine B) are provided as aqueous solutions. The concentrations of prepolymer A) and/or polyamine B), expressed as solids contents, may independently be in a range of ≥ 5 weight-% to ≤ 20 weight-%, based on the total weight of the solution.

In another embodiment the aqueous solution of prepolymer A) and/or the aqueous solution of polyamine B) have a viscosity of ≤ 50 mPas at 23 °C as determined according to DIN EN ISO 3219 Preferred are viscosities of ≥ 1 mPas to ≤ 20 mPas, more preferred ≥ 2 mPas to ≤ 10 mPas.

In another embodiment the water C) comprises a base, an acid or a pH buffer. The presence of a base or a buffer has the advantage of countering acid formation if the water comes into contact with acid-forming gases such as carbon dioxide. Carbon dioxide may by employed as a carrier gas for spraying the hydrogel-forming composition. It is noted that carbon dioxide is readily available in many operating theaters. Preferred bases are ammonia, trisodium phosphate, disodium hydrogenphosphate, tripotassium phosphate, dipotassium hydrogenphosphate, sodium borate, sodium hydroxide, potassium hydroxide, sodium carbonate, sodium bicarbonate, potassium carbonate, potassium bicarbonate or a mixture of at least two of the aforementioned bases. The presence of an acid or a buffer has the advantage that a rise in pH on the surface of, for example, an internal organ to which is in contact with the hydrogel is limited. Preferably the pH does not rise over 8 after 30 minutes following the application of the hydrogel. Buffer concentrations are preferred in the range of ≥ 0% to ≤ 50% H⁺ with regard to free amine groups calculated according to equation 1.4. Preferably *f_{buffering grade amine}* is in the range of 0 to 0.5, more preferred in the range of 0 to 0.4 and even more preferred 0 to 0.25. ${f}_{buffering grade amine} = \frac{{V}_{solution} ∗ {M}_{acidic component}}{\frac{{V}_{solution} ∗ {δ}_{solution} ∗ \frac{solid {content}_{amine}}{100}}{eq . {weight}_{amine}}}$

| | |
|---|---|
| *M_{acidic component}=* Mol/l of acidic component | *f*_{*buffering grade* amine}= grade of buffered free amine groups in the range of 0.0-1.0 |
| *Vₛₒₗᵤₜᵢₒₙ=* Volume of amine component | *eq. weightₘᵢₙₑ* = Equivalent weight amine as determined |
| *δₛₒₗᵤₜᵢₒₙ=* density of the solution (for all conducted experiments =1) | 100= factor for percentage |

In another embodiment the diisocyanate A1) is an aliphatic diisocyanate. Preferred examples include pentamethylene diisocyanate, hexamethylene diisocyanate, isophorone diisocyanate and H12-MDI.

In another embodiment the prepolymer A) has an NCO group content of ≥ 1 weight-% to ≤ 10 weight-% as determined according to DIN EN ISO 11909. Preferred are ≥ 2 weight-% to ≤ 4 weight-%.

In another embodiment the polyamine B) is an amino group-terminated polyether. Such polyether amines may comprise primary amino groups attached to polyether backbones, typically based on propylene oxide, ethylene oxide or a mixture of both oxides. Commercially available examples include those found in the Jeffamine product families. Preferred are trimethylolpropane- (TMP) or glycerol- (glycerin) started amino group-terminated polyoxpropylenes, commercial examples of which can be found in the Jeffamine T series. Other preferred polyether amines are polyether diamines with more polyoxypropylene groups than polyoxyethylene groups in the backbone, commercial examples of which can be found in the Jeffamine ED series. Specific examples are Jeffamine ED2003 and Jeffamine T403 as described in the experimental section of this disclosure.

In another embodiment the equivalent weights of the prepolymer A) and the polyamine B) have a ratio of ≥ 0.25 to ≤ 4. Preferably the equivalent weight ratios are ≥ 0.5 to ≤ 2, more preferred ≥ 1.5 to ≤ 1.7. This facilitates spraying applications of aqueous solutions of prepolymer A) and polyamine B).

In another embodiment aqueous solutions of prepolymer A) and polyamine B) are mixed in a mixing or spraying head and the resulting mixture is applied onto a target surface, thereby forming the hydrogel on the target surface. The target surface may be an internal organ during surgery.

In another embodiment the method further comprises adding as component D) an active pharmaceutical ingredient to a precursor of the hydrogel and/or to the hydrogel itself.

Suitable active pharmaceutical ingredients include:
1. Anti-infectives, such as antibiotics, including penicillin, tetracycline, chlortetracycline bacitracin, nystatin, streptomycin, neomycin, polymyxin, gramicidin, oxytetracycline, chloramphenicol, and erythromycin; sulfonamides, including sulfacetamide, sulfamethazine, sulfadiazine, sulfamerazine, sulfamethizole and sulfisoxazole; antivirals, including idoxuridine; and other anti-infectives including nitrofurazone and sodium propionate.
2. Anti-allergenics such as antazoline, methapyrilene, chlorpheniramine, pyrilamine and prophenpyridamine.
3. Anti-inflammatories such as hydrocortisone, cortisone, dexamethasone 21-phosphate, fluocinolone, triamcinolone, medrysone, prednisolone, prednisolone 21-phosphate, and prednisolone acetate.
4. Decongestants such as phenylephrine, naphazoline, and tetrahydrazoline.
5. Miotics and antichlolinesterases such as pilocarpine, eserine salicylate, carbachol, diisopropyl fluorophosphate, phospholine iodide, demecarium bromide, physostigmin and neostigmin.
6. Mydriatics such as atropine sulfate, cyclopentolate, homatropine, scopolamine, tropicamide, eucatropine, and hydroxyamphetamine.
7. Sympathomimetics such as epinephrine.
8. Neuroleptics such as reserpine and chlorpromazine.
9. Estrogens such as estrone, 17*β*-estradiol, ethinyl estradiol, and diethyl stilbesterol.
10. Progestational agents such as progesterone, 19-norprogesterone, norethindrone, megestrol, melengestrol, chlormadinone, ethisterone, medroxyprogesterone, norethynodrel and 17a-hydroxy-progesterone.
11. Humoral agents such as the prostaglandins, for example, PGE1, PGE2, and PGF2.
12. Antipyretics such as ibuprofen, acetylsalicylic acid, sodium salicylate, and salicylamide.
13. Antispasmodics such as atropine, methantheline, papaverine, and methscopolamine bromide.
14. Cardioactive agents such as benzydroflumethiazide, flumethiazide, chlorothiazide, and aminotrate.

One type of preferred pharmaceutical compounds is a progestogen or a drug having a progestogenic activity. In particular the compound may be selected from the group of progesterone and its derivatives, cyproterone acetate, desogestrel, etonogestrel, levonorgestrel, lynestrenol, medroxyprogesterone acetate, norethisterone, norethisterone acetate, norgestimate, drospirenone, gestodene, 19-nor-17-hydroxy progesterone esters, 17α-ethinyltestosterone and derivatives thereof, 17a-ethinyl-19-nor-testosterone and derivatives thereof, ethynodiol diacetate, dydrogesterone, norethynodrel, allylestrenol, medrogestone, norgestrienone, ethisterone, dl-norgestrel or a mixture of at least two of the aforementioned compounds.

Another type of preferred pharmaceutical compound is a drug capable of preventing or suppressing endometrial bleeding. In particular the compound may be selected from the group of prostaglandin synthesis inhibitors, NSAIDs, inhibitors of leukotriene, oxytocin antagonists, pancreatic trypsin inhibitors, COX-inhibitors, antifibrinolytic drugs, estrogens, antiestrogens, aromatase inhibitors, cytokine inhibitors, glucocorticoids, progestogens with pronounced glucocorticoid acticity, danazol, gestrinone, angiogenesis inhibitors or a mixture of at least two of the aforementioned compounds.

It is possible to combine the progestogen or a drug having a progestogenic activity with the drug capable of preventing or suppressing endometrial bleeding. Particularly preferred is the combination of levonorgestrel with tranexamic acid, mefenamic acid, danazol or an angiogenesis inhibitor. This is useful in an intrauterine device.

Another type of preferred pharmaceutical compound is a calcium channel blocker, in particular nimodipine or nifedipine.

Another type of preferred pharmaceutical compound is a cytostatic agent such as cisplatin, rituximab, bevacizumab, trastuzumab, imatinib, lenalidomide, pemetrexed, bortezomib, cetuximab, leuprolein or abiraterone.

Lastly, opioid analgetics and non-opioid analgetics are also a type of preferred pharmaceutical compound.

The pharmaceutical compounds (drugs) can be in different forms, such as uncharged molecules, components of molecular complexes, or non-irritating, pharmacologically acceptable salts such as hydrochloride, hydrobromide, sulphate, phosphate, nitrate, borate, acetate, maleate, tartrate, salicylate, etc. For acidic drugs, salts of metals, amines, or organic cations (e.g., quaternary ammonium) can be employed. Furthermore, simple derivatives of the drugs (such as ethers, esters, amides, etc.) which have desirable retention and release characteristics but which are easily hydrolyzed by body pH, enzymes, etc., can be employed.

The hydrogel according to the invention can be used as a delayed-release formulation for the pharmaceutical compound, pesticide, herbicide, pheromone or combination of at least two of the aforementioned compounds distributed within the hydrogel.

**In** its use as a delayed release formulation the hydrogel can be viewed as a drug delivery device or system. Drug delivery systems of the invention can take a wide variety of shapes and forms for administering the drugs at controlled rates to different areas of the body. Thus, the invention includes external and internal drug-delivery systems such as skin patches, sublingual or buccal tablets, peroral dosage forms, implants for releasing a drug in the tissues of a living organism, pessaries, prosthesis, artificial glands, vaginal or rectal suppositories, cervical rings, troches, drug-dispensing intrauterine devices and ocular inserts.

Another aspect of the invention is a hydrogel obtainable by a method according to the present invention.

Another aspect of the invention is a hydrogel obtainable by a method according to the present invention for use in a method of surgery.

Another aspect of the invention is a hydrogel obtainable by a method according to the present invention for use as a post-surgical adhesion barrier. The prevention of intraabdominal adhesions is expressly contemplated.

The specification also discloses a device comprising a first storage volume, a second storage volume and a mixer in fluid communication with the first and second storage volume, wherein the mixer is configured to mix the contents of the first and second storage volume and to discharge the resulting mixture from the device, wherein the first storage volume comprises the prepolymer A) as recited in connection with the method according to the present invention, the second storage volume comprises the polyamine B) as recited in connection with the method according to the present invention. The first and/or second storage volumes may further comprise water. It is also possible that the device comprises a third storage volume which comprises water. The water may be added to the prepolymer A) and/or polyamine B) prior to their mixing.

Preferably the equivalent weights of the prepolymer A) and the polyamine B) employed in the device, have a ratio of ≥ 0.25 to ≤ 4. Preferably the equivalent weight ratios are ≥ 0.5 to ≤ 2, more preferred ≥ 1.5 to ≤ 1.7. This facilitates spraying applications of aqueous solutions of prepolymer A) and polyamine B).

The present invention shall be further described with reference to the following examples without wishing to be limited by them.

### Experimental section:

Apparatus and Analytical Methods Used:
pH-Meter: Knick Portamess 911 pH,
Viscosity determination: DIN EN ISO 3219
Residual monomer content determination: DIN EN ISO 10283
Hydroxyl number determination: DIN EN ISO 4629-2
Acidic number determination: DIN EN ISO 2114
Amine number determination: DIN EN ISO 1877-2
NCO content determination: DIN EN 1242
Equivalent weight determination: The equivalent weight (eq. weight) was determined according to the following equation: $eq . weight = \frac{42 ∗ 100}{NCO content %}$

**Table 1: Raw materials used for the experiments**

| **Name** | **CAS** | **Purity** | **Supplier** |
|---|---|---|---|
| **Jeffamines** | | | |
| Jeffamine ED2003 | 65605-36-9 | n.d. | Huntsman |
| Jeffamine T403 | 39423-51-3 | n.d. | Huntsman |

| **Diisocyanates** | | | |
|---|---|---|---|
| Hexamethylene diisocyanate | 822-06-0 | n.d. | Covestro AG |

| **Starters and monomers for polyol synthesis** | | | |
|---|---|---|---|
| Polyether 1 | - | n.d. | Covestro AG |
| Polyether 2 | - | n.d. | Covestro AG |
| Ethylene oxide | 75-21-8 | n.d. | GHC Gerling Holz |
| Propylene oxide | 75-56-9 | n.d. | Chemogas |
| Dilactide | 95-96-5 | >99% | Sigma-Aldrich |

| **Polyols** | | | |
|---|---|---|---|
| Polyether 3 | - | n.d. | Covestro AG |

| **Catalysts** | | | |
|---|---|---|---|
| dibutyl phosphate | 107-66-4 | ≥ 97.0% | Sigma-Aldrich |

| **Buffer agents** | | | |
|---|---|---|---|
| HCl 0.1M | 7647-01-0 | n.d. | Honeywell Fluka |
| Sodium acetate | 127-09-3 | ACS reagent, ≥ 99.0% | Sigma-Aldrich |
| Acetic acid, glacial | 64-19-7 | ACS reagent, ≥ 99.7% | Sigma-Aldrich |
| Sodium chloride | 7647-14-5 | +80 mesh, ≥ 98% | Sigma-Aldrich |
| Potassium chloride | 7447-40-7 | ACS reagent, 99.0-100.5% | Sigma-Aldrich |
| Potassium phosphate monobasic, anhydrous | 7778-77-0 | ACS reagent, ≥ 99.0% | Sigma-Aldrich |
| di-Sodium hydrogen phosphate heptahydrate | 7782-85-6 | EMPROVE^{®} EXPERT, DAC, USP | Merck |
| Sodium dihydrogen phosphate monohydrate | 10049-21-5 | EMPROVE^{®} EXPERT, BP, USP | Merck |
| NaOH 0.1M | 1310-73-2 | n.d. | Honeywell Fluka |

Jeffamine ED2003 was an *O,O'*-bis(2-aminopropyl) polypropylene glycol-*block*-polyethylene glycol-*block*-polypropylene glycol having a molecular weight of ca. 2000 g/mol and the idealized formula CH₃CH(NH₂)CH₂[OCH(CH₃)CH₂]ₗ(OCH₂CH₂)ₘ[OCH₂CH(CH₃)]ₙNH₂ with m being ca. 39 and (l+n) being ca. 6.

Jeffamine T403 was a trimethylolpropane tris[poly(propylene glycol), amine terminated] ether having a molecular weight of ca. 440 g/mol and the idealized formula C₂H₅C[CH₂[OCH₂CH(CH₃)]ₙNH₂]₃ with n being 5 to 6.

### Preparation of amine solutions

For the preparation of the amine solution, which was used for the preparation of the hydrogels, either Jeffamine ED2003, predominantly based on PEG, functionality=2, equivalent weight=1021 g/eq. (Huntsman) or Jeffamine T403, predominantly based on PEG, functionality=3, equivalent weight=81 g/eq. (Huntsman), were used. Solutions were prepared in aqueous media using deionized H₂O and/or buffers or aqueous acid like phosphate (monobasic/dibasic), acetic acid/acetate buffer, HCl. Composition of the prepared solutions is stated in table 2.

### Preparation of hydrogels:

### Without carrier gas:

Two cylinders were used for the application. One with a volume of 25 ml (Isocyanate component) and one with a volume of 12.5 ml (amine component). For the mixing process a static mixer MAH 4.0-17S from AdChem was used. Both components were dispensed in a constant volume ratio of 2:1 (isocyanate:amine) within 25 seconds.

### With carrier gas carbon dioxide (CO₂)

Two cylinders were used for the application. One with a volume of 10 ml (Isocyanate component) and one with a volume of 5 ml (amine component). For the mixing process a spray applicator from Baxter was used. Both components were dispensed in a constant volume ratio of 2:1 (isocyanate:amine) within 30 seconds with a constant gasflow of 2.0-2.5 NL/min CO₂.

### Determination of pH-values

pH-measurements were conducted by spraying both components into a beaker with a pH electrode inside. pH-measurements were conducted for 30 minutes.

This applies for all herein stated examples, except example 35. Carbon dioxide supported spraying leads to a falsification of the pH values since carbon dioxide is solved in the aqueous solution in the beaker as consequence of the spraying process. To avoid falsification pH paper stripes (1-14, Macherey-Nagel Tritest) have been used for the determination of pH values of formulations using CO₂ as carrier gas.

### Determination of adhesion on organs:

A kidney (pig) preheated to 37 °C was used as a substrate for the application. Hydrogels were prepared as described above. The following rating system may be used to evaluate the adhesion of each formulation to the kidney.

| **Rating Score** | **Adhesion Properties** |
|---|---|
| 1 | No adhesion |
| 2 | Very poor adhesion (hydrogel can be removed without applying mechanical force, e.g., by movement of the kidney) |
| 3 | Poor adhesion (hydrogel can be removed by applying light mechanical force, like wiping over the surface with a moistened gloves) |
| 4 | Good adhesion (hydrogel can be removed by applying stronger mechanical force, like pulling or rubbing) |
| 5 | Very good adhesion (hydrogel cannot be removed as a whole by applying strong mechanical force, like pulling or rubbing) |

### Determination of biodegradability via loss of mechanical properties:

For the determination of the biodegradation rate hydrogels were prepared as described above but dispensed into 3.9 mL PE molds (thick end of 2 ml type pipettes, Vₜₒₜₐₗ=5.9 ml by VITLAB^{®}). After curing for 5 min they were weighed and transferred into a 25 mL glass bottle. 22 ml PBS-solution (pH=7.33) were added and the glass bottles were stored at 37 °C in an incubator with shaking speed of 60 rpm. Every 24 h the hydrogel samples were taken out of the buffer solution to record the weight. Afterwards, the old buffer solution was replaced with fresh buffer solution and the samples were again stored in the incubator. This procedure was repeated every 24 hours and the samples were incubated until they were fully dissolved in the medium. The point at which the hydrogels could not be removed from the glass bottles without disintegration into smaller gel particles is described as "Loss of mechanical properties" (table 4). Without wishing to be bound by theory it is assumed that there would be no barrier effect of the hydrogel anymore, so this point in time is regarded as an end point.

### Determination of curing time

To determine the curing time of each hydrogel formulation, the mixtures were sprayed on a vertical release paper and the time the mixed solutions needed for the gelation process was measured. The curing time was defined as the point where no more liquids ran from the release paper and the droplets began to form a hydrogel according to visual observation.

### Spray experiment example:

Application example (cf. example 13):
Provision of the amine component:

For a preparation of 12.5 ml of amine solution which was used in a ratio of 1/1.2 (NH₂/NCO groups), 1.82 g of Jeffamine ED2003 were weighed into a flask and added up to 12.50 g with aqueous HCl (0.0260M). Afterwards, the solution was stirred and then filled into a cylinder of a spray applicator.

### Provision of the isocyanate component:

3.75 g of prepolymer 4 were weighed into a mixing cup and filled up to 25.00 g with demineralized H₂O. The solution was mixed for 30 seconds. Afterwards the solution was filled into a cylinder of a spray applicator and the spray experiments were conducted immediately (within 15 seconds).

### Polyol synthesis

### Polyol 1

1645.00 g of a glycerin (F=3) started polyoxypropylene triol (Polyether 1; Covestro Charge 1712041028) with an OH-number of 233 mg KOH/g, 83.50 g of dilactide (Aldrich, LOT BCBV8611) and 2.00 g of a DMC-catalyst (prepared as stated in WO 01/80994 A1, example 6) were weighed into a 20l stainless-steel pressure reactor under nitrogen atmosphere. After stripping with nitrogen for 30 min. at 100 °C and 0.1 bar, the temperature of the reactor was set to 130 °C. 2215.00 g of propylene oxide (Chemogas) and 6058.00 g of ethylene oxide (GHC Gerling Holz) were dosed within 135 min. to the reaction mixture and were reacted for further 45 min at 130 °C. Afterwards the reaction was cooled to 90 °C and volatile compounds were distilled off for 30 min. at <0.01 bar. The reaction mixture was stabilized with 6.00 g of Irganox 1076.
Ester group content expressed as -C(=O)-O-: 0.51 wt.-%
Ethylene oxide content: 60.57 wt.-%
OH number: 39.90 mg KOH/g
Acid number: 0.01 mg KOH/g
Viscosity: 1010 mPa*s, 25 °C
PO/EO/dilactide amount ratios: 26.5%/72.5%/1%

### Polyol 2

1645.00 g of a glycerin (F=3) started polyoxypropylene triol (Polyether 1; Covestro Charge 1712041028) with an OH-number of 233 mg KOH/g, 209.00 g of dilactide (Aldrich, LOT BCBV8611) and 2.00 g of a DMC-catalyst (prepared as stated in WO 01/80994 A1, example 6) were weighed into a 20l stainless-steel pressure reactor under nitrogen atmosphere. After stripping with nitrogen for 30 min. at 100 °C and 0.1 bar, the temperature of the reactor was set to 130 °C. 2069.00 g of propylene oxide (Chemogas) and 6058.00 g of ethylene oxide (GHC Gerling Holz) were dosed within 135 min. to the rection mixture and were reacted for further 45 min at 130 °C. Afterwards the reaction was cooled to 90 °C and volatile compounds were distilled off for 30 min. at <0.01 bar. The reaction mixture was stabilized with 6.00 g of Irganox 1076.
Ester group content expressed as -C(=O)-O-: 1.28 wt.-%
Ethylene oxide content: 60.70 wt.-%
OH number: 39.7 mg KOH/g
Acid number: 0.01 mg KOH/g
Viscosity: 1040 mPa*s, 25 °C
PO/EO/dilactide amount ratios: 25.0%/72.5%/2.5%

### Polyol 3

1194.70 g of a glycerin (F=3) started polyoxypropylene triol (Polyether 1; Covestro Charge 1712041028) with an OH-number of 233 mg KOH/g, 300.70 g of dilactide (Aldrich, LOT BCBV8611) and 0.75 g of a DMC-catalyst (prepared as stated in WO 01/80994 A1, example 6) were weighed into a 20l stainless-steel pressure reactor under nitrogen atmosphere. After stripping with nitrogen for 30 min. at 100 °C and 0.1 bar, the temperature of the reactor was set to 130 °C and the pressure to 2.21 bar with nitrogen. 1353.40 g of propylene oxide (Chemogas) and 4361.00 g of ethylene oxide (GHC Gerling Holz) were dosed within 135 min. to the rection mixture and were reacted for further 45 min at 130 °C. Afterwards the reaction was cooled to 90 °C and volatile compounds were distilled off for 30 min. at <0.01 bar. The reaction mixture was stabilized with 6.00 g of Irganox 1076.
Ester group content expressed as -C(=O)-O- 2.56 wt.-%
Ethylene oxide content: 60.48 wt.-%
OH number: 38.5 mg KOH/g
Acid number: 0.02 mg KOH/g
Viscosity: 1145 mPa*s, 25 °C
PO/EO/dilactide amount ratios (wt.%): 22.5%/72.5%/5%

### Polyol 4

1643.00 g of a glycerin (F=3) started polyoxypropylene triol (Polyether 1; Covestro Charge 1712041028) with an OH-number of 233 mg KOH/g, 833.0 g of dilactide (Aldrich, LOT BCBV8611) and 2.0 g of a DMC-catalyst (prepared as stated in WO 01/80994 A1, example 6) were weighed into a 20l stainless-steel pressure reactor under nitrogen atmosphere. After stripping with nitrogen for 30 min. at 100 °C and 0.1 bar, the temperature of the reactor was set to 130 °C. 1450.00 g of propylene oxide (Chemogas) and 6058.00 g of ethylene oxide (GHC Gerling Holz) were dosed within 135 min. to the rection mixture and were reacted for further 45 min at 130 °C. Afterwards the reaction was cooled to 90 °C and volatile compounds were distilled off for 30 min. at <0.01 bar. The reaction mixture was stabilized with 6.00 g of Irganox 1076.
Ester group content expressed as -C(=O)-O-: 5.09 wt.-%
Ethylene oxide content: 60.68 wt.-%
OH number: 37.20 mg KOH/g
Acid number: 0.02 mg KOH/g
Viscosity: 1400 mPa*s, 25 °C
PO/EO/dilactide amount ratios: 17.5%/72.5%/10%

### Polyol 5

1425.00 g of a 1,2-propane diol (F=2) started polyoxypropylene diol (Polyether 2; Covestro Charge 1712041028) with an OH-number of 233 mg KOH/g, 428.00 g of dilactide (Aldrich, LOT BCBV8611) and 2.00 g of a DMC-catalyst (prepared as stated in WO 01/80994 A1, example 6) were weighed into a 20l stainless-steel pressure reactor under nitrogen atmosphere.

After stripping with nitrogen for 30 min. at 100 °C and 0.1 bar, the temperature of the reactor was set to 130 °C. 1929.40 g of propylene oxide (Chemogas) and 6216.90 g of ethylene oxide (GHC Gerling Holz) were dosed within 135 min. to the rection mixture and were reacted for further 45 min at 130 °C. Afterwards the reaction was cooled to 90 °C and volatile compounds were distilled off for 30 min. at <0.01 bar. The reaction mixture was stabilized with 6.00 g of Irganox 1076.
Ester group content expressed as -C(=O)-O-: 2.61 wt.-%
Ethylene oxide content: 62.17 wt.-%
OH number: 37.50 mg KOH/g
Acid number: 0.20 mg KOH/g
Viscosity: 802 mPa*s, 25 °C
PO/EO/dilactide amount ratios: 22.5%/72.5%/5%

### Prepolymer Synthesis:

For the prepolymer synthesis all used polyethers were dehydrated at 120°C and 20 mbar for 2 hours.

### Prepolymer 1

592.00 g of hexamethylene diisocyanate (HDI) and 0.65 g dibutyl phosphate (DBP) were weighed into a three-neck flask and heated up to 80 °C under constant stirring. 697.50 g of polyether 3 (F=3, OHZ=37, Starter (Glycerin): 1.96%; EO: 70.89%, PO: 26.43%) , were added dropwise within 2 hours. After the addition was completed, the reaction was stirred for one additional hour until the residual NCO content reached 21.46%. The reaction mixture was allowed to cool down under constant stirring. Unreacted monomer was removed by thin film evaporation (p=0.1 mbar; Tₚᵣₑ₋ₑᵥₐₚₒᵣₐₜₒᵣ=130 °C, Tₘₐᵢₙ₋ₑᵥₐₚₒᵣₐₜₒᵣ=120 °C) and an NCO-terminated prepolymer with NCO-content= 2.49 wt. % was obtained.
Ester group content expressed as -C(=O)-O-: 0 wt.-%
Residual Monomer content: 0.03 wt.-%
NCO content: 2.49 wt.-%
Viscosity: 3950 mPa*s, 23 °C
Average equivalent weight: 1686.75 g/eq.

### Prepolymer 2

628.05 g of hexamethylene diisocyanate (HDI) and 0.66 g dibutyl phosphate (DBP) were weighed into a three-neck flask and heated up to 80 °C under constant stirring under nitrogen atmosphere. 700.00 g of polyol 1 were added dropwise within 2 hours. After the addition was completed, the reaction was stirred for one additional hour until residual NCO content reached 22.06%.. The reaction mixture was allowed to cool down under constant stirring. Unreacted monomer was removed by thin film evaporation (p=0.1 mbar; Tₚᵣₑ₋ₑᵥₐₚₒᵣₐₜₒᵣ=130 °C, Tₘₐᵢₙ₋ₑᵥₐₚₒᵣₐₜₒᵣ=120 °C) and an NCO-terminated prepolymer with NCO content= 2.63 wt. % was obtained.
Ester group content expressed as -C(=O)-O-: 0.46 wt.-%
Residual Monomer content: 0.03 wt.-%
NCO content: 2.63 wt.-%
Viscosity: 3967 mPa*s, 23°C
Average equivalent weight: 1596.96 g/eq.

### Prepolymer 3

625.64 g of hexamethylene diisocyanate (HDI) and 0.66 g dibutyl phosphate (DBP) were weighed into a three-neck flask and heated up to 80 °C under constant stirring under nitrogen atmosphere. 700.00 g of polyol 2, were added dropwise within 2 hours. After the addition was completed, the reaction was stirred for one additional hour until residual NCO content reached 19.41%. The reaction mixture was allowed to cool down under constant stirring. Unreacted monomer was removed by thin film evaporation (p=0.1 mbar; Tₚᵣₑ₋ₑᵥₐₚₒᵣₐₜₒᵣ=130 °C, Tₘₐᵢₙ₋ₑᵥₐₚₒᵣₐₜₒᵣ=120 °C) and an NCO-terminated prepolymer with NCO content= 2.67 wt. % was obtained.
Ester group content expressed as -C(=O)-O-: 1.14 wt.-%
Residual Monomer content: 0.02 wt.-%
NCO content: 2.67 wt.-%
Viscosity: 4451 mPa*s, 23°C
Average equivalent weight: 1573.03 g/eq.

### Prepolymer 4

592.00 g of hexamethylene diisocyanate (HDI) and 0.61 g dibutyl phosphate (DBP) were weighed into a three-neck flask and heated up to 80 °C under constant stirring. 632.92 g of polyol 3 were added dropwise within 2 hours. After the addition was completed, the reaction was stirred for one additional hour until residual NCO content reached 22.56%. The reaction mixture was allowed to cool down under constant stirring. Unreacted monomer was removed by thin film evaporation (p=0.1 mbar; Tₚᵣₑ₋ₑᵥₐₚₒᵣₐₜₒᵣ=130 °C, Tₘₐᵢₙ₋ₑᵥₐₚₒᵣₐₜₒᵣ=120 °C) and an NCO-terminated prepolymer with NCO content= 2.55 wt. % was obtained.
Ester group content expressed as -C(=O)-O-: 2.29 wt.-%
Residual Monomer content: >0.01 wt.-%
NCO content: 2.53 wt.-%
Viscosity: 3990 mPa*s, 23 °C
Average equivalent weight: 1647.06 g/eq.

### Prepolymer 5

592.00 g of hexamethylene diisocyanate (HDI) and 0.65 g dibutyl phosphate (DBP) were weighed into a three-neck flask and heated up to 80 °C under constant stirring. 703.92 g of polyol 4 were added dropwise within 2 hours. After the addition was completed, the reaction was stirred for one additional hour until residual NCO content reached 21.30%. The reaction mixture was allowed to cool down under constant stirring. Unreacted monomer was removed by thin film evaporation (p=0.1 mbar; Tₚᵣₑ₋ₑᵥₐₚₒᵣₐₜₒᵣ=130 °C, Tₘₐᵢₙ₋ₑᵥₐₚₒᵣₐₜₒᵣ=120 °C) and an NCO-terminated prepolymer with NCO content= 2.59 wt. % was obtained.
Ester group content expressed as -C(=O)-O-: 4.58 wt.-%
Residual Monomer content: 0.03 wt.-%
NCO content: 2.59 wt.-%
Viscosity: 9451 mPa*s, 23 °C
Average equivalent weight: 1621.62 g/eq.

### Prepolymer 6

458.80 g of hexamethylene diisocyanate (HDI) and 0.50 g dibutyl phosphate (DBP) were weighed into a three-neck flask and heated up to 80 °C under constant stirring. 541.2 g of polyol 5 were added dropwise within 2 hours. After the addition was completed, the reaction was stirred for one additional hour until residual NCO content reached 21.39%. The reaction mixture was allowed to cool down under constant stirring. Unreacted monomer was removed by thin film evaporation (p=0.1 mbar; Tₚᵣₑ₋ₑᵥₐₚₒᵣₐₜₒᵣ=130 °C, Tₘₐᵢₙ₋ₑᵥₐₚₒᵣₐₜₒᵣ=120 °C) and an NCO-terminated prepolymer with NCO content= 2.23 wt. % was obtained.
Ester group content expressed as -C(=O)-O-: 2.35 wt.-%
Residual Monomer content: 0.02 wt.-%
NCO content: 2.23 wt.-%
Viscosity: 3648 mPa*s, 23 °C
Average equivalent weight: 1883.41 g/eq.

**Table 2: Composition of isocyanate and amine solutions used for the preparation of different hydrogels.**

| Comparative examples are denoted as "(c)". Functionalities of the prepolymer and amine are denoted as "F(prep.)" and "F(amine)", respectively. Viscosity is stated in millipascal*seconds and is listed as "[mPa*s]". The solid content of the used Isocyanate or amine component is given in weight% and is stated as "[wt.-%]". The molar concentrations of the basic and acidic fractions are denoted as "[M]". | | | | | | | | | | | | |
|---|---|---|---|---|---|---|---|---|---|---|---|---|
| | **Isocyanate component** | | | | **Amine component (Jeffamine ED2003.** | | | | | | | |
| Ex. | Prepolymer used | F (prep.) | Solid content of the prepared isocyanate solution [wt.-%] | Viscosity of the obtained isocyanate solution at 21 °C [mPa*s] | Amine used | F (amine) | Solid content of the prepared amine solution [wt.-%] | Buffer/ Acid type used as solvent | Concent ration of basic fraction in the buffer [M] | Concentra tion of acidic fraction in the buffer [M] | Viscosity of the obtained amine solution at 23 °C [mPa*s] | Molar ratio NH₂/ NCO |
| 1 (c) | 4 | >2 | 10% | 3.68 | Jeffamine ED2003 | 2 | 11.71% | H₂O | - | - | 2.53 | 1:1 |
| 2a (c) | 1 | >2 | 10% | 3.48 | Jeffamine ED2003 | 2 | 11.71% | Phosphate buffer | 0.0173 | 0.0173 | 2.69 | 1:1 |
| 2b (c) | 1 | >2 | 10% | 3.48 | Jeffamine ED2003 | 2 | 11.71% | Phosphate buffer | 0.02882 | 0.02882 | 3.22 | 1:1 |
| 2c (c) | 1 | >2 | 10% | 3.48 | Jeffamine ED2003 | 2 | 11.71% | Phosphate buffer | 0.0404 | 0.0404 | 3.68 | 1:1 |
| 2d (c) | 1 | >2 | 10% | 3.48 | Jeffamine ED2003 | 2 | 11.71% | Phosphate buffer | 0.0577 | 0.0577 | 2.95 | 1:1 |
| 3a (c) | 1 | >2 | 10% | 3.48 | Jeffamine ED2003 | 2 | 11.71% | Acetate buffer | 0.0173 | 0.0173 | 3.07 | 1:1 |
| 3b (c) | 1 | >2 | 10% | 3.48 | Jeffamine ED2003 | 2 | 11.71% | Acetate buffer | 0.02882 | 0.02882 | 4.22 | 1:1 |
| 3c (c) | 1 | >2 | 10% | 3.48 | Jeffamine ED2003 | 2 | 11.71% | Acetate buffer | 0.0404 | 0.0404 | 3.79 | 1:1 |
| 3d (c) | 1 | > 2 | 10% | 3.48 | Jeffamine ED2003 | 2 | 11.71% | Acetate buffer | 0.0669 | 0.0669 | 2.83 | 1:1 |
| 4a (c) | 1 | >2 | 10% | 3.48 | Jeffamine ED2003 | 2 | 11.71% | HCl solution | - | 0.005775 | 2.11 | 1:1 |
| 4b (c) | 1 | > 2 | 10% | 3.48 | Jeffamine ED2003 | 2 | 11.71% | HCl solution | - | 0.0173 | 2.35 | 1:1 |
| 4c (c) | 1 | > 2 | 10% | 3.48 | Jeffamine ED2003 | 2 | 11.71% | HCl solution | - | 0.02882 | 2.09 | 1:1 |
| 4d (c) | 1 | > 2 | 10% | 3.48 | Jeffamine ED2003 | 2 | 11.71% | HCl solution | - | 0.0404 | 2.53 | 1:1 |
| 5 | 5 | > 2 | 11,625% | 4.9 | Jeffamine ED2003 | 2 | 12.20% | Acetate | 0.0299 | 0,0299 | 3.04 | 1:1.2 |
| 6 | 5 | > 2 | 15% | 4.35 | Jeffamine ED2003 | 2 | 15.74% | HCl | - | 0.0276 | 3.58 | 1:1.2 |
| 7 | 5 | > 2 | 15% | 4.35 | Jeffamine ED2003 | 2 | 15.74% | Acetate | 0.0283 | 0.0283 | 4.64 | 1:1.2 |
| 8 | 5 | > 2 | 15% | 4.35 | Jeffamine ED2003 | 2 | 15.74% | HCl | - | 0.0386 | 3.69 | 1:1.2 |
| 9 (c) | 4 | > 2 | 11,625% | 6.56 | Jeffamine ED2003 | 2 | 11.26% | HCl | - | 0.0276 | 3.54 | 1:1.2 |
| 10 | 4 | > 2 | 15% | 8.39 | Jeffamine ED2003 | 2 | 14.53% | HCl | - | 0.0260 | 2.74 | 1:1.2 |
| 11 | 5 | > 2 | 15% | 4.35 | Jeffamine ED2003 | 2 | 15.74% | HCl | - | 0.0213 | 3.82 | 1:1.2 |
| 12 | 4 | > 2 | 12.95% | 7.48 | Jeffamine ED2003 | 2 | 12.54% | Acetate | 0.0256 | 0.0256 | 2.34 | 1:1.2 |
| 13 | 4 | > 2 | 15% | 8.39 | Jeffamine ED2003 | 2 | 14.53% | HCl | - | 0.0260 | 3.55 | 1:1.2 |
| 14 (c) | 5 | > 2 | 10% | 3.30 | Jeffamine ED2003 | 2 | 10.50% | HCl | - | 0.0257 | 2.65 | 1:1.2 |
| 15 (c) | 5 | > 2 | 10% | 3.30 | Jeffamine ED2003 | 2 | 10.50% | Acetate | 0.0154 | 0.0154 | 3.37 | 1:1.2 |
| 16 | 4 | > 2 | 15% | 8.39 | Jeffamine ED2003 | 2 | 14.53% | Acetate | 0.0356 | 0.0356 | 4.65 | 1:1.2 |
| 17 | 5 | > 2 | 12.787% | 4.48 | Jeffamine ED2003 | 2 | 13.43% | Acetate | 0.0197 | 0.0197 | 3.47 | 1:1.2 |
| 18 | 4 | > 2 | 13.375% | 8.51 | Jeffamine ED2003 | 2 | 12.95% | Acetate | 0.0191 | 0.0191 | 2.47 | 1:1.2 |
| 19 | 5 | > 2 | 14.25% | 6.82 | Jeffamine ED2003 | 2 | 14.96% | Acetate | 0.0346 | 0.0346 | 4.85 | 1:1.2 |
| 20 (c) | 2 | >2 | 10% | 3.80 | Jeffamine ED2003 | 2 | 10.66% | HCl | - | 0,02609 | 2.64 | 1:1.2 |
| 21 | 3 | >2 | 12.5% | 4.82 | Jeffamine ED2003 | 2 | 13.52% | HCl | - | 0,01987 | 3.26 | 1:1.2 |
| 22 (c) | 3 | >2 | 10% | 3.11 | Jeffamine ED2003 | 2 | 10.82% | Acetate | 0,02649 | 0,02649 | 2.42 | 1:1.2 |
| 23 | 3 | >2 | 11.25% | 4.51 | Jeffamine ED2003 | 2 | 12.17% | Acetate | 0,02384 | 0,02384 | 2.57 | 1:1.2 |
| 24 (c) | 2 | >2 | 15% | 7.21 | Jeffamine ED2003 | 2 | 15.98% | Acetate | 0,02348 | 0,02348 | 4.88 | 1:1.2 |
| 25 (c) | 3 | >2 | 10% | 3.11 | Jeffamine ED2003 | 2 | 10.82% | Acetate | 0,02649 | 0,02649 | 2.35 | 1:1.2 |
| 26 (c) | 3 | >2 | 10% | 3.11 | Jeffamine ED2003 | 2 | 10.82% | HCl | - | 0,02119 | 2.57 | 1:1.2 |
| 27 (c) | 2 | >2 | 15% | 7.21 | Jeffamine ED2003 | 2 | 15.98% | Acetate | 0,02348 | 0,02348 | 4.12 | 1:1.2 |
| 28 (c) | 2 | >2 | 12.5% | 4.87 | Jeffamine ED2003 | 2 | 13.32% | HCl | - | 0,01957 | 3.19 | 1:1.2 |
| 29 (c) | 2 | >2 | 15% | 7.21 | Jeffamine ED2003 | 2 | 15.98% | HCl | - | 0,03914 | 3.68 | 1:1.2 |
| 30 (c) | 2 | >2 | 10% | 3.80 | Jeffamine ED2003 | 2 | 10.66% | HCl | - | 0,02087 | 2.68 | 1:1.2 |
| 31 (c) | 6 | 2 | 10.8% | 3.74 | Jeffamine T403 | 3 | 1.55% | Acetate | 0.0265 | 0.0265 | 1.11 | 1:1.2 |
| 32 (c) | 6 | 2 | 13.5% | 4.72 | Jeffamine T403 | 3 | 1.94% | HCl | - | 0.020 | 1.18 | 1:1.2 |
| 33 | 6 | 2 | 16.25% | 6.97 | Jeffamine T403 | 3 | 2.33% | H₂O | - | - | 1.30 | 1:1.2 |
| 34 (c) | 6 | 2 | 11.17% | 3.74 | Jeffamine ED2003 | 2 | 12.11% | Acetate | 0.01857 | 0.01857 | 2.81 | 1:1 |
| 35 | 3 | >2 | 15% | 7.21 | Jeffamine ED2003 | 2 | 14.58% | NaOH | 0.0200 | - | 4.58 | 1:1.2 |

**Table 3: Solid content of the obtained hydrogels based on formulations given in table 3.**

| Comparative examples are denoted as "(c)". | | | | | | | | | | |
|---|---|---|---|---|---|---|---|---|---|---|
| Example | 1 (c) | 2a (c) | 2b (c) | 2c (c) | 2d (c) | 3a (c) | 3b (c) | 3c (c) | 3d (c) | 4a (c) |
| Solids content [weight-%] | 10.57 | 10.57 | 10.57 | 10.57 | 10.57 | 10.57 | 10.57 | 10.57 | 10.57 | 10.57 |
| Example | 4b (c) | 4c (c) | 4d (c) | 5 | 6 | 7 | 8 | 9 (c) | 10 | 11 |
| Solids content [weight-%] | 10.57 | 10.57 | 10.57 | 11.73 | 14.74 | 14.74 | 14.74 | 11.50 | 14.84 | 14.74 |
| Example | 12 | 13 | 14 (c) | 15 (c) | 16 | 17 | 18 | 19 | 20 (c) | 21 |
| Solids content [weight-%] | 12.81 | 14.84 | 10.09 | 10.16 | 14.84 | 13.00 | 13.23 | 14.49 | 10.22 | 12.84 |
| Example | 22 (c) | 23 | 24 (c) | 25 (c) | 26 (c) | 27 (c) | 28 (c) | 29 (c) | 30 (c) | 31 (c) |
| Solids content [weight-%] | 10.27 | 11.56 | 15.33 | 10.27 | 10.27 | 15.33 | 12.77 | 15.33 | 10.22 | 7.72 |
| Example | 32 (c) | 33 | 34 (c) | 35 | | | | | | |
| Solids content [weight-%] | 9.65 | 11.61 | 11.48 | 14.86 | | | | | | |

**Table 4: Overview on results of important parameters for an adhesion prevention.**

| Comparative examples are denoted as "(c)" and "n. a." denotes that the property in question has not been analyzed. For the purposes of the present invention the following thresholds were applied to denote the suitability of the hydrogel: loss of mechanical properties: < 28 days; adhesion on organs: 2; 3; 4 or 5; curing time: ≤ 120 seconds. | | | | | | |
|---|---|---|---|---|---|---|
| The adhesion on organs was rated on a scale of 1 to 5 according to the score previously discussed with 1 denoting the poorest and 5 the best adhesion. | | | | | | |
| Example | pH after 30 min. | Loss of mechanical properties [d] | Elongation [%] | Tensile strength [kPa] | Adhesion on organs | Curing time [s] |
| 1 (c) | 9.84 | n. a. | n. a. | n. a. | 1 | 15 |
| 2a (c) | 8.72 | n. a. | n. a. | n. a. | 1 | 20 |
| 2b (c) | 8.14 | n. a. | n. a. | n. a. | 1 | 20 |
| 2c (c) | 7.84 | n. a. | n. a. | n. a. | 1 | 20 |
| 2d (c) | 7.51 | n. a. | n. a. | n. a. | 1 | 120 |
| 3a (c) | 8.61 | n. a. | n. a. | n. a. | 1 | 20 |
| 3b (c) | 8.9 | n. a. | n. a. | n. a. | 1 | 20 |
| 3c (c) | 6.65 | n. a. | n. a. | n. a. | 1 | 150 |
| 3d (c) | 6.46 | n. a. | n. a. | n. a. | 1 | No curing observed |
| 4a (c) | 9.44 | n. a. | n. a. | n. a. | 1 | 20 |
| 4b (c) | 6.76 | n. a. | n. a. | n. a. | 1 | 20 |
| 4c (c) | 6.78 | n. a. | n. a. | n. a. | 1 | 20 |
| 4d (c) | 6.71 | n. a. | n. a. | n. a. | 1 | 20 |
| 5 | 7.3 | 0.33 | 128 | 11.27 | 2 | 29 |
| 6 | 6.65 | 3 | 89 | 12.78 | 5 | 24 |
| 7 | 6.82 | 6 | 70 | 16.69 | 5 | 19 |
| 8 | 6.7 | 3 | 116 | 12.87 | 4 | 18 |
| 9 (c) | 6.65 | 7 | 258 | 32.33 | 1 | 24 |
| 10 | 7.86 | 13 | 190 | 32.9 | 4 | 11 |
| 11 | 6.92 | 3 | 174 | 43.2 | 4 | 18 |
| 12 | 7.08 | 8 | 278 | 92.5 | 2 | 13 |
| 13 | 6.59 | 13 | 256 | 63.4 | 4 | 9 |
| 14 (c) | 6.7 | 2 | 257 | 57.2 | 1 | 64 |
| 15 (c) | 7.47 | 1.33 | 88 | 7.7 | 1 | 50 |
| 16 | 6.52 | 9 | 319 | 72.6 | 4 | 22 |
| 17 | 7.81 | 6 | 121 | 28.6 | 3 | 27 |
| 18 | 6.74 | 12 | 214 | 67.9 | 4 | 9 |
| 19 | 7.17 | 6 | 96 | 17.3 | 5 | 12 |
| 20 (c) | 6.67 | 1 | 311.14 | 15.52 | 1 | 360 |
| 21 | 6.7 | 11 | 143.2 | 34.68 | 2 | 16 |
| 22 (c) | 6.62 | 10 | 273.75 | 62.27 | 1 | 30 |
| 23 | 6.63 | 10 | 223.65 | 18.13 | 3 | 17 |
| 24 (c) | 6.76 | >28 | 243.36 | 61.71 | 5 | 7 |
| 25 (c) | 6.74 | 10 | 191.07 | 13.62 | 1 | 29 |
| 26 (c) | 6.72 | 9 | 239.39 | 20.82 | 1 | 22 |
| 27 (c) | 6.73 | >28 | 169.81 | 39.39 | 4 | 7 |
| 28 (c) | 6.69 | >28 | 273.48 | 44.44 | 5 | 13 |
| 29 (c) | 6.88 | >28 | 380.56 | 52.3 | 5 | 9 |
| 30 (c) | 6.63 | >28 | 172.12 | 16.56 | 1 | 30 |
| 31 (c) | n. a. | n. a. | n. a. | n. a. | n. a. | No curing observed |
| 32 (c) | n. a. | n. a. | n. a. | n. a. | n. a. | No curing observed |
| 33 | n. a. | n. a. | 240.96 | 41.58 | n. a. | 23 |
| 34 (c) | n. a. | n. a. | n. a. | n. a. | n. a. | No curing observed |
| 35 | 7 | n. a. | n. a. | n. a. | 5 | 40 |

## Claims

1. A method of manufacturing a hydrogel comprising reacting the components:
A) an isocyanate-terminated prepolymer obtained by reacting a mixture comprising:
A1) a diisocyanate with a molar weight of 140 to 278 g/mol;
A2) a polyalkylene oxide with an ethylene oxide content of ≥ 50 weight-%, calculated from the total weight of the polyalkylene oxide;
B) a polyamine having a molecular weight, determined according to the description, of ≥ 200 g/mol, preferably the polyamine B) has a molecular weight of ≥ 200 g/mol and ≤ 10000 g/mol, more preferably the polyamine B) has a molecular weight of ≥ 300 g/mol and ≤ 10000 g/mol;
C) water;
**characterized in that**
the polyalkylene oxide A2) and/or the polyamine B) further comprise ester groups, expressed as -C(=O)-O- groups, in an amount of > 0.51 weight-%, preferably in an amount of > 0.51 weight-% to ≤ 5.1 weight-%, more preferably in an amount of > 0.51 weight-% to ≤ 5 weight-%, even more preferably in an amount of > 0.51 weight-% to ≤ 4.5 weight-%, most preferably in an amount of ≥ 1 weight-% to ≤ 4.5 weight-%, especially in an amount of ≥ 1.2 weight-% to ≤ 3 weight-% based on the total weight of polyalkylene oxide A2) or polyamine B), respectively,
the average OH functionality of polyalkylene oxide A2) is greater than 2 and/or the average combined primary and secondary amine functionality of polyamine B) is greater than 2,
the combined components A) and B) are present in an amount of > 11.5 weight-%, preferably in an amount of > 11.5 weight-% to ≤ 50 weight-%, more preferably in an amount of > 13 weight-% to ≤ 40 weight-%, even more preferably in an amount of > 13.2 weight-% to ≤ 20 weight-% based on the total weight of components A), B) and C).

2. The method according to claim 1, **characterized in that** the polyamine B) does not comprise ester groups, expressed as -C(=O)-O- groups, and the polyalkylene oxide A2) further comprises ester groups, expressed as -C(=O)-O- groups, in an amount of > 0.51 weight-%, preferably in an amount of > 0.51 weight-% to ≤ 5.1 weight-%, more preferably in an amount of > 0.51 weight-% to ≤ 5 weight-%, even more preferably in an amount of > 0.51 weight-% to ≤ 4.5 weight-%, most preferably in an amount of ≥ 1 weight-% to ≤ 4.5 weight-%, especially in an amount of ≥ 1.2 weight-% to ≤ 3 weight-% based on the total weight of polyalkylene oxide A2).

3. The method according to any one of the preceding claims, wherein the NCO/NH molar ratio between prepolymer A) and polyamine B) is > 1.0 to ≤ 1.4, preferably the NCO/NH molar ratio between prepolymer A) and polyamine B) is ≥ 1.1 to ≤ 1.3.

4. The method according to any one of the preceding claims, wherein the prepolymer A) and/or the polyamine B) are provided as aqueous solutions.

5. The method according to claim 4, wherein the aqueous solution of prepolymer A) and/or the aqueous solution of polyamine B) have a viscosity of ≤ 50 mPas at 23 °C as determined according to DIN EN ISO 3219.

6. The method according to any one of the preceding claims, wherein the water C) comprises a base, an acid or a pH buffer.

7. The method according to any one of the preceding claims, wherein the diisocyanate A1) is an aliphatic diisocyanate.

8. The method according to any one of the preceding claims, wherein the prepolymer A) has an NCO group content of ≥ 1 weight-% to ≤ 10 weight-%, preferably the prepolymer A) has an NCO group content of ≥ 2 weight-% to ≤ 4 weight-% as determined according to DIN EN ISO 11909.

9. The method according to any one of the preceding claims, wherein the polyamine B) is an amino group-terminated polyether.

10. The method according to any one of the preceding claims, wherein the equivalent weights of the prepolymer A) and the polyamine B) have a ratio of ≥ 0.25 to ≤ 4, preferably the equivalent weights of the prepolymer A) and the polyamine B) have a ratio of ≥ 0.5 to ≤ 2, more preferably the equivalent weights of the prepolymer A) and the polyamine B) have a ratio of ≥ 1.5 to ≤ 1.7.

11. The method according to any one of claims 4 to 10, wherein aqueous solutions of prepolymer A) and polyamine B) are mixed in a mixing or spraying head and the resulting mixture is applied onto a target surface, thereby forming the hydrogel on the target surface.

12. A hydrogel obtainable by a method according to any one of the preceding claims.

13. Use of a hydrogel according to claim 12 in a method of surgery.

14. Use of a hydrogel according to claim 12 as a post-surgical adhesion barrier.

15. Use of a hydrogel according to claim 12 in delayed-release formulations.

## Patentansprüche

1. Verfahren zur Herstellung eines Hydrogels, umfassend das Umsetzen der folgenden Komponenten:
A) ein isocyanat-terminiertes Präpolymer, erhältlich durch Umsetzen eines Gemisches, umfassend:
A1) ein Diisocyanat mit einem Molgewicht von 140 bis 278 g/mol;
A2) ein Polyalkylenoxid mit einem Ethylenoxidgehalt von ≥ 50 Gew.-%, berechnet aus dem Gesamtgewicht des Polyalkylenoxids;
B) ein Polyamin mit einem Molekulargewicht, gemäß der Beschreibung bestimmt, von ≥ 200 g/mol, vorzugsweise weist das Polyamin B) ein Molekulargewicht von ≥ 200 g/mol und ≤ 10000 g/mol auf, bevorzugter weist das Polyamin B) ein Molekulargewicht von ≥ 300 g/mol und ≤ 10000 g/mol auf;
C) Wasser;
**dadurch gekennzeichnet, dass**
das Polyalkylenoxid A2) und/oder das Polyamin B) ferner Estergruppen umfasst/umfassen, ausgedrückt als -C(=O)-O-Gruppen, in einer Menge von > 0.51 Gew.-%, vorzugsweise in einer Menge von > 0.51 Gew.-% bis ≤ 5.1 Gew.-%, bevorzugter in einer Menge von > 0.51 Gew.-% bis ≤ 5 Gew.-%, noch bevorzugter in einer Menge von > 0.51 Gew.-% bis ≤ 4.5 Gew.-%, am meisten bevorzugt in einer Menge von ≥ 1 Gew.-% bis ≤ 4.5 Gew.-%, insbesondere in einer Menge von ≥ 1.2 Gew.-% bis ≤ 3 Gew.-%, bezogen auf das Gesamtgewicht des Polyalkylenoxids A2) bzw. des Polyamins B),
wobei die durchschnittliche OH-Funktionalität des Polyalkylenoxids A2) größer als 2 ist und/oder die durchschnittliche kombinierte primäre und sekundäre Aminfunktionalität des Polyamins B) größer als 2 ist, wobei die kombinierten Komponenten A) und B) in einer Menge von > 11.5 Gew.-%, vorzugsweise in einer Menge von > 11.5 Gew.-% bis ≤ 50 Gew.-%, bevorzugter in einer Menge von > 13 Gew.-% bis ≤ 40 Gew.-%, noch bevorzugter in einer Menge von > 13.2 Gew.-% bis ≤ 20 Gew.-%, bezogen auf das Gesamtgewicht der Komponenten A), B) und C), vorliegen.

2. Verfahren nach Anspruch 1, **dadurch gekennzeichnet, dass** das Polyamin B) keine Estergruppen, ausgedrückt als -C(=O)-O-Gruppen, umfasst, und das Polyalkylenoxid A2) ferner Estergruppen, ausgedrückt als -C(=O)-O-Gruppen, in einer Menge von > 0.51 Gew.-%, vorzugsweise in einer Menge von > 0.51 Gew.-% bis ≤ 5.1 Gew.-%, bevorzugter in einer Menge von > 0.51 Gew.-% bis ≤ 5 Gew.-%, noch bevorzugter in einer Menge von > 0.51 Gew.-% bis ≤ 4.5 Gew.-%, am meisten bevorzugt in einer Menge von ≥ 1 Gew.-% bis ≤ 4.5 Gew.-%, insbesondere in einer Menge von ≥ 1.2 Gew.-% bis ≤ 3 Gew.-%, bezogen auf das Gesamtgewicht des Polyalkylenoxids A2), umfasst.

3. Verfahren nach einem der vorhergehenden Ansprüche, wobei das NCO/NH-Molverhältnis zwischen Präpolymer A) and Polyamin B) > 1.0 bis ≤ 1.4 beträgt, vorzugsweise das NCO/NH-Molverhältnis zwischen Präpolymer A) und Polyamin B) ≥ 1.1 bis ≤ 1.3 beträgt.

4. Verfahren nach einem der vorhergehenden Ansprüche, wobei das Präpolymer A) und/oder das Polyamin B) als wässrige Lösungen bereitgestellt werden.

5. Verfahren nach Anspruch 4, wobei die wässrige Lösung von Präpolymer A) und/oder die wässrige Lösung von Polyamin B) eine Viskosität von ≤ 50 mPas bei 23 °C, wie gemäß DIN EN ISO 3219 bestimmt, aufweisen.

6. Verfahren nach einem der vorhergehenden Ansprüche, wobei das Wasser C) ein Base, eine Säure oder einen pH-Puffer umfasst.

7. Verfahren nach einem der vorhergehenden Ansprüche, wobei es sich bei dem Diisocyanat A1) um ein aliphatisches Diisocyanat handelt.

8. Verfahren nach einem der vorhergehenden Ansprüche, wobei das Präpolymer A) einen NCO-Gruppengehalt von ≥ 1 Gew.-% bis ≤ 10 Gew.-% aufweist, das Präpolymer A) vorzugsweise einen NCO-Gruppengehalt von ≥ 2 Gew.-% bis ≤ 4 Gew.-%, wie gemäß DIN EN ISO 11909 bestimmt, aufweist.

9. Verfahren nach einem der vorhergehenden Ansprüche, wobei es sich bei dem Polyamin B) um einen Aminogruppen-terminierten Polyether handelt.

10. Verfahren nach einem der vorhergehenden Ansprüche, wobei das Äquivalentgewicht des Präpolymers A) und des Polyamins B) ein Verhältnis von ≥ 0.25 bis ≤ 4 aufweist, das Äquivalentgewicht des Präpolymers A) und des Polyamins B) vorzugsweise ein Verhältnis von ≥ 0.5 bis ≤ 2 aufweist, das Äquivalentgewicht des Präpolymers A) und des Polyamins B) bevorzugter ein Verhältnis von ≥ 1.5 bis ≤ 1.7 aufweist.

11. Verfahren nach einem der Ansprüche 4 bis 10, wobei wässrige Lösungen von Präpolymer A) und Polyamin B) in einem Misch- oder Sprühkopf vermischt werden und das resultierende Gemisch auf die Zieloberfläche aufgetragen wird, wodurch sich das Hydrogel auf der Zieloberfläche bildet.

12. Hydrogel, erhältlich durch ein Verfahren nach einem der vorhergehenden Ansprüche.

13. Verwendung eines Hydrogels nach Anspruch 12 in einem chirurgischen Verfahren.

14. Verwendung eines Hydrogels nach Anspruch 12 als eine postoperative Adhäsionsbarriere.

15. Verwendung eines Hydrogels nach Anspruch 12 in Formulierungen mit verzögerter Freisetzung.

## Revendications

1. Procédé de fabrication d'un hydrogel comprenant la réaction des composants suivants :
A) un prépolymère à terminaison isocyanate obtenu par réaction d'un mélange comprenant :
A1) un diisocyanate ayant une masse molaire de 140 à 278 g/mol ;
A2) un poly(oxyde d'alkylène) ayant une teneur en oxyde d'éthylène ≥ 50 % en poids, calculée par rapport au poids total du poly(oxyde d'alkylène) ;
B) une polyamine ayant une masse moléculaire, déterminée selon la description, ≥ 200 g/mol, de préférence la polyamine B) a une masse moléculaire ≥ 200 g/mol et ≤ 10000 g/mol, plus préférentiellement la polyamine B) a une masse moléculaire ≥ 300 g/mol et ≤ 10000 g/mol ;
C) de l'eau ;
**caractérisé en ce que**
le poly(oxyde d'alkylène) A2) et/ou la polyamine B) comprennent en outre des groupes ester, exprimés en groupes -C(=O)-O-, à une teneur > 0.51 % en poids, de préférence à une teneur > 0.51 % en poids et ≤ 5.1 % en poids, plus préférentiellement à une teneur > 0.51 % en poids et ≤ 5 % en poids, encore plus préférentiellement à une teneur > 0.51 % en poids et ≤ 4.5 % en poids, tout particulièrement de préférence à une teneur ≥ 1 % en poids et ≤ 4.5 % en poids, en particulier à une teneur ≥ 1.2 % en poids et ≤ 3 % en poids par rapport au poids total de poly(oxyde d'alkylène) A2) ou de polyamine B), respectivement,
le nombre moyen de groupes OH du poly(oxyde d'alkylène) a2) est supérieur à 2 et/ou le nombre moyen combiné de groupes amine primaires et secondaires de la polyamine B) est supérieur à 2,
les composants A) et B) combinés sont présents à une teneur > 11.5 % en poids, de préférence à une teneur > 11.5 % en poids et ≤ 50 % en poids, plus préférentiellement à une teneur > 13 % en poids et ≤ 40 % en poids, encore plus préférentiellement à une teneur > 13.2 % en poids et ≤ 20 % en poids par rapport au poids total des composants A), B) et C).

2. Procédé selon la revendication 1, **caractérisé en ce que** la polyamine B) ne comprend pas de groupes ester, exprimés en groupes -C(=O)-O-, et le poly(oxyde d'alkylène) A2) comprend en outre des groupes ester, exprimés en groupes -C(=O)-O-, à une teneur > 0.51 % en poids, de préférence à une teneur > 0.51 % en poids et ≤ 5.1 % en poids, plus préférentiellement à une teneur > 0.51 % en poids et ≤ 5 % en poids, encore plus préférentiellement à une teneur > 0.51 % en poids et ≤ 4.5 % en poids, tout particulièrement de préférence à une teneur ≥ 1 % en poids et ≤ 4.5 % en poids, en particulier à une teneur ≥ 1.2 % en poids et ≤ 3 % en poids par rapport au poids total de poly(oxyde d'alkylène) A2).

3. Procédé selon l'une quelconque des revendications précédentes, dans lequel le rapport molaire NCO/NH du prépolymère A) à la polyamine B) est > 1.0 et ≤ 1.4, de préférence le rapport molaire NCO/NH du prépolymère A) à la polyamine B) est ≥ 1.1 et ≤ 1.3.

4. Procédé selon l'une quelconque des revendications précédentes, dans lequel le prépolymère A) et/ou la polyamine B) sont fournis sous forme de solutions aqueuses.

5. Procédé selon la revendication 4, dans lequel la solution aqueuse de prépolymère A) et/ou la solution aqueuse de polyamine B) ont une viscosité ≤ 50 mPa.s à 23 °C, déterminée selon la norme DIN EN ISO 3219.

6. Procédé selon l'une quelconque des revendications précédentes, dans lequel l'eau C) comprend une base, un acide ou un tampon de pH.

7. Procédé selon l'une quelconque des revendications précédentes, dans lequel le diisocyanate A1) est un diisocyanate aliphatique.

8. Procédé selon l'une quelconque des revendications précédentes, dans lequel le prépolymère A) a une teneur en groupes NCO ≥ 1 % en poids et ≤ 10 % en poids, de préférence le prépolymère A) a une teneur en groupes NCO ≥ 2 % en poids et ≤ 4 % en poids, déterminée selon la norme DIN EN ISO 11909.

9. Procédé selon l'une quelconque des revendications précédentes, dans lequel la polyamine B) est un polyéther à terminaison amino.

10. Procédé selon l'une quelconque des revendications précédentes, dans lequel les poids équivalents du prépolymère A) et de la polyamine B) ont un rapport ≥ 0.25 et ≤ 4, de préférence les poids équivalents du prépolymère A) et de la polyamine B) ont un rapport ≥ 0.5 et ≤ 2, plus préférentiellement les poids équivalents du prépolymère A) et de la polyamine B) ont un rapport ≥ 1.5 et ≤ 1.7.

11. Procédé selon l'une quelconque des revendications 4 à 10, dans lequel les solutions aqueuses de prépolymère A) et de polyamine B) sont mélangées dans une tête de mélange ou de pulvérisation et le mélange obtenu est appliqué sur une surface cible, formant ainsi l'hydrogel sur la surface cible.

12. Hydrogel pouvant être obtenu par un procédé selon l'une quelconque des revendications précédentes.

13. Utilisation d'un hydrogel selon la revendication 12 dans une méthode chirurgicale.

14. Utilisation d'un hydrogel selon la revendication 12 en tant que barrière contre les adhérences postopératoires.

15. Utilisation d'un hydrogel selon la revendication 12 dans des formulations à libération retardée.
